# EUROPÄISCHE PATENTSCHRIFT

(11) **EP 2 877 442 B1**
(45) Veröffentlichungstag und Bekanntmachung des Hinweises auf die Patenterteilung: **16.11.2016**
(21) Anmeldenummer: 13739716.2
(22) Anmeldetag: 23.07.2013
(51) Int. Cl.: C07C 201/08

(54) **VERFAHREN ZUR HERSTELLUNG VON NITROBENZOL DURCH ADIABATE NITRIERUNG**
METHOD FOR PRODUCING NITROBENZENE BY ADIABATIC NITRIDING
PROCÉDÉ DE PREPARATION DE NITROBENZÈNE PAR NITRIFICATION ADIABATIQUE

(30) Priorität: 27.07.2012 EP 12178161
(43) Veröffentlichungstag der Anmeldung: 03.06.2015
(73) Patentinhaber: Covestro Deutschland AG, 51373 Leverkusen (DE)
(72) Erfinder: KNAUF, Thomas, 41542 Dormagen (DE); MERKEL, Michael, 40223 Düsseldorf (DE)
(74) Vertreter: Levpat
(86) Internationale Anmeldenummer: PCT/EP2013/065506
(87) Internationale Veröffentlichungsnummer: WO 2014/016292

(56) Entgegenhaltungen:
- EP-A1- 2 246 320
- DE-A1-102009 005 324

## Beschreibung

Gegenstand der Erfindung ist ein kontinuierliches Verfahren zur Herstellung von Nitrobenzol durch Nitrierung von Benzol mit Mischungen aus Schwefel- und Salpetersäure unter Einsatz eines stöchiometrischen Überschusses an Benzol, bei dem der Gehalt an aliphatischen organischen Verbindungen im Einsatzbenzol während der Anfahrzeit der Produktionsanlage stets kleiner als 1,5 Massen-%, bezogen auf die Gesamtmasse des Einsatzbenzols, gehalten wird. Dies wird erreicht, indem entweder während der Anfahrzeit das Einsatzbenzol aus rezykliertem nicht umgesetzten Benzol (Rückbenzol) und der Reaktion neu zugeführtem Benzol (Frischbenzol), abhängig von der Reinheit beider Ströme, in entsprechenden Mengenverhältnissen gemischt wird, oder indem während der Anfahrzeit auf die Rezyklierung nicht umgesetzten Benzols komplett verzichtet wird, das Einsatzbenzol also nur aus der Reaktion neu zugeführtem Benzol besteht.

Die vorliegende Erfindung betrifft ein kontinuierliches Verfahren zur Herstellung von Nitrobenzol durch adiabate Nitrierung von Benzol durch ein Gemisch von Schwefel- und Salpetersäure (sog. Mischsäure). Ein solches Verfahren wurde erstmals in US 2,256,999 beansprucht und ist in moderneren Ausführungsformen in US 4,091,042, US 5,313,009 und US 5,763,697 beschrieben.

Den beschriebenen adiabaten Verfahren ist gemein, dass die Ausgangsstoffe Benzol und Salpetersäure in einem großen Überschuss an Schwefelsäure zur Reaktion gebracht werden, welche die frei werdende Reaktionswärme und das bei der Reaktion gebildete Wasser aufnimmt.

Die Reaktionsführung erfolgt im Allgemeinen so, dass die Salpetersäure und Schwefelsäure zur sogenannten Nitriersäure (auch Mischsäure genannt) vereint werden. Benzol wird in diese Nitriersäure hinein dosiert. Die Reaktionsprodukte sind im Wesentlichen Wasser und Nitrobenzol. In der Nitrierreaktion wird Benzol bezogen auf die molare Salpetersäuremenge mindestens in stöchiometrischer Menge, aber bevorzugt in 2%igem bis 10%igem Überschuss eingesetzt. Das in den Reaktionsapparaten gebildete und im Phasentrennapparat von der Säurephase abgetrennte Rohnitrobenzol wird gemäß dem Stand der Technik einer Wäsche und einer destillativen Aufarbeitung unterzogen, wie beispielsweise in EP 1 816 117 A1 (Seite 2, Zeile 26 bis 42), US 4,091,042 (siehe oben) oder US 5,763,697 (siehe oben) beschrieben. Charakteristisch für diese Aufarbeitung ist, dass nicht umgesetztes überschüssiges Benzol nach der Wäsche von Nitrobenzol in einer abschließenden Destillation abgetrennt wird und als Rückbenzol, das auch niedrig siedende, nicht-aromatische organische Verbindungen (sog. Leichtsieder) enthält (siehe DE 10 2009 005 324 A1), wieder in der Nitrierreaktion eingesetzt wird. Die Behandlung des Abgases aus der adiabaten Nitrierreaktion wird in EP 0 976 718 B1 beschrieben. Das Abgas aus Säurekreislauf und fertigem Rohnitrobenzol wird abgezogen, vereint und über einen NOx-Absorber geschickt, um verdünnte Salpetersäure zurückzugewinnen, die in die Reaktion zurückgefahren wird. Die als Kreislaufsäure bezeichnete Schwefelsäure wird in einem Blitzverdampfer aufkonzentriert und weitestgehend von Organika befreit. Es verbleiben hochsiedende Organika wie z. B. Nitrobenzol, Dinitrobenzol und Nitrophenole in Spuren in der Kreislaufsäure und werden somit auch zur Reaktion zurückgefahren.

Die Güte eines adiabaten Verfahrens zur Nitrierung aromatischer Kohlenwasserstoffe ist einerseits definiert durch den Gehalt des Produktes an unerwünschten Nebenprodukten der Reaktion, die durch mehrfache Nitrierung oder Oxidation des aromatischen Kohlenwasserstoffes oder des Nitroaromaten gebildet werden. Das Bestreben bei der Herstellung von Nitrobenzol ist es, den Gehalt an Dinitrobenzol und an Nitrophenolen, insbesondere des als brisant einzustufenden Trinitrophenols (Pikrinsäure), zu minimieren. Andererseits ist die Güte eines adiabaten Verfahrens dadurch definiert, dass der Prozess ohne technischen Produktionsausfall betrieben werden kann.

Um Nitrobenzol mit besonders hohen Selektivitäten zu erhalten, wurde die Art der zu verwendenden Mischsäure detailliert festgelegt (EP 0 373 966 B1, EP 0 436 443 B1 und EP 0 771 783 B1), sowie darauf hingewiesen, dass der Gehalt an Nebenprodukten durch die Höhe der Maximaltemperatur bestimmt wird (EP 0 436 443 B1, Spalte 15, Z. 22 bis 25). Auch ist bekannt, dass ein hoher Anfangsumsatz vorteilhaft ist für eine hohe Selektivität und dass diese dann erreicht wird, wenn zu Beginn der Reaktion eine optimale Durchmischung herbeigeführt wird (EP 0 771 783 B1, Absatz [0014]).

Zu hervorragenden Selektivitäten gelangt man, wenn die Reaktionsstarttemperatur sehr niedrig gewählt wird (WO2010/051616 A1), was aber gleichbedeutend mit einer mehrfach verlängerten Reaktionszeit ist. Eine hohe Raum-Zeit-Ausbeute ist für die industrielle Anwendung eines Verfahrens von Vorteil, da dadurch kompakte Reaktionseinrichtungen gebaut werden können, die sich durch ein geringes Investitionsvolumen pro Kapazität auszeichnen. Daher ist diese Vorgehensweise kontraproduktiv.

Allen aufgeführten Literaturstellen gemein ist, dass sie den Anfahrprozess einer Nitrieranlage und seine Schwierigkeiten nicht beschreiben.

Bezüglich der Qualität des Einsatzstoffes Benzol auf die adiabate Herstellung von Nitrobenzol beschreibt EP 2 246 320 A1, dass handelsübliches Benzol je nach Quelle mehr oder weniger stark verunreinigt sein kann. Typische Verunreinigungen sind andere Aromaten, insbesondere Toluol und Xylol, die in Benzol gängiger Reinheit bis zu jeweils 0,05 Massen-% enthalten sein können. Andere für Benzol typische Verunreinigungen sind nichtaromatische organische Verbindungen, die in Summe bis zu 0,07 Massen-% ausmachen können. Insbesondere sind hier Cyclohexan (bis zu 0,03 Massen-%) und Methylcyclohexan (bis zu 0,02 Massen-%) genannt. Die vorstehend beschriebenen Verunreinigungen stören die nachfolgenden Schritte in der Prozesskette zur Herstellung von Di- und Polyisocyanaten der Diphenylmethanreihe (MDI) in den genannten Konzentrationen entweder überhaupt nicht oder nur geringfügig, beispielsweise durch eine minimale Erschwerung der Abwasser- und Abluftaufbereitung im Nitrobenzolverfahren durch nichtaromatische Organika im Benzol. Eine aufwändige Reinigung des Benzols für den Einsatz in der MDI-Prozesskette wird daher für unverhältnismäßig erachtet und kann unterbleiben. In EP 2 246 320 A1 wird nicht auf die nichtaromatischen organischen Verbindungen im nach erfolgter Reaktion vom Rohprodukt abgetrennten und in die Nitrierung zurückgeführten Benzol (sog. "Rückbenzol") eingegangen.

DE 10 2009 005 324 A1 offenbart, dass technisches Benzol üblicherweise einen Anteil von leichtsiedenden nichtaromatischen organischen Verbindungen (Leichtsieder) von 0,01 bis 0,5 % hat. In den gängigen Verfahren der Benzolnitrierung wird jedoch nicht technisches Benzol als solches eingesetzt, sondern eine Mischung aus Rückbenzol und technischem Benzol, sodass der Gehalt an Leichtsiedern im tatsächlich eingesetzten Benzol beträchtlich höher sein kann als in handelsüblichem technischem Benzol. DE 10 2009 005 324 A1 offenbart beispielhaft einen Wert von 5 % (Abschnitt [0007]). Gemäß der Lehre dieser Schrift ist ein solch hoher Leichtsiederanteil in der eigentlichen Nitrierung noch nicht nachteilig. DE 10 2009 005 324 A1 geht nur auf Probleme bei der sich anschließenden Phasentrennung ein (Abschnitt [0008]). Zur Lösung dieser Probleme wird ein spezielles Phasentrennverfahren unter Einsatz eines Druckhalte-Siphons vorgeschlagen.

EP 2 155 655 B1 geht nur auf aromatische Verunreinigungen (Alkyl-substituierte Aromaten) im Benzol ein.

DE-AS-1 129 466 beschreibt ein Verfahren zum Mononitrieren von technischem Benzol, Xylol und Toluol, die die üblichen Gehalte an aliphatischen Kohlenwasserstoffen aufweisen, bei dem der in der Destillation des Nitroaromaten anfallende Vorlauf an nicht umgesetztem Aromaten, der reich an aliphatischen Verunreinigungen ist, mit frischem Aromaten vermischt, der Nitrierung zuführt und den erneut anfallenden Vorlauf beliebig oft im Kreis führt. (Im Falle von Benzol als Ausgangstoff entspricht der Aromatenvorlauf dieser Schrift dem oben erwähnten Rückbenzol.) Der Fachmann entnimmt dieser Schrift mithin die technische Lehre, dass ein erhöhter Anteil an aliphatischen Verunreinigungen im einzusetzenden Aromaten die Nitrierung nicht stört.

Den beschriebenen Verfahren des Standes der Technik gelingt es zwar, ein Nitrobenzol herzustellen, das einen geringen Gehalt an Nebenprodukten aufweist, also nur von etwa 100 ppm bis 300 ppm an Dinitrobenzol und 1500 ppm bis 2500 ppm an Nitrophenolen enthält, wobei Pikrinsäure einen Anteil von 10 Massen-% bis 50 Massen-% der Nitrophenole annehmen kann. Die Verfahren zeichnen sich auch durch eine hohe Raum-/Zeitausbeute aus. Es werden jedoch nur Verfahren beschrieben, die schon in Gang sind, d. h., bei denen der Zeitraum von Beginn der Reaktion bis zum Erreichen der Soll-Last (sog. "Anfahrzeit") bereits verstrichen ist. Evtl. besondere Schwierigkeiten beim Anfahren eines adiabaten Nitrierprozesses werden nicht angesprochen.

Ausgangspunkt für die vorliegende Erfindung war die Erkenntnis, dass Verunreinigungen in den Ausgangsstoffen, insbesondere Verunreinigungen im eingesetzten Benzol (d. i. nach dem Stand der Technik ein Gemisch aus frischem Benzol technischer Reinheit und Rückbenzol), sich während der Anfahrzeit in besonderem Maße negativ auf das Verfahren auswirken.

Verunreinigungen im frisch zugeführten Benzol und/oder im Rückbenzol erniedrigen die insgesamt verfügbare Konzentration an Benzol. Hierdurch wird die Reaktion verlangsamt, was sich während der Anfahrzeit, wenn die endgültige Reaktionstemperatur noch nicht erreicht und dadurch die Reaktionsgeschwindigkeit gegenüber dem Zustand einer eingefahrenen Anlage ohnehin erniedrigt ist, besonders kritisch auswirkt. Die verringerte Benzolkonzentration kann zur Folge haben, dass eine zu große Menge an Salpetersäure eingesetzt wird. Dies wiederum erhöht die Menge an unerwünschten mehrfach nitrierten Produkten und NOx-Gasen. Letzteres hat die Bildung weiterer Nebenprodukte zur Folge. In diesem Sinne kritische Verunreinigungen sind insbesondere aliphatische organische Verbindungen (Leichtsieder, siehe oben). Diese können während der Reaktion gemeinsam mit NOx-Gasen ausgasen und dadurch weitere Nachteile hervorrufen wie bspw. eine schlechtere Durchmischung der Reaktanden und ein reduziertes Reaktionsvolumen.

Dem vorstehend Gesagten Rechnung tragend ist ein Gegenstand der vorliegenden Erfindung ein Verfahren zur Herstellung von Nitrobenzol, bei dem besonderes Augenmerk auf den kritischen Zeitraum des Anfahrens der Reaktion gelegt wird. Insbesondere wurde gefunden, dass durch eine Beschränkung des Gehaltes an aliphatischen organischen Verbindungen im tatsächlich eingesetzten Benzol mindestens während der Anfahrzeit die zuvor genannten Schwierigkeiten überwunden oder zumindest deutlich vermindert werden. Diese Beschränkung des Gehaltes an aliphatischen organischen Verbindungen im tatsächlich eingesetzten Benzol mindestens während der Anfahrzeit ist auf verschiedene Weisen realisierbar, die Gegenstände der vorliegenden Erfindung sind.

Insbesondere ist ein Gegenstand der vorliegenden Erfindung ein kontinuierliches Verfahren zur Herstellung von Nitrobenzol durch Nitrierung von Benzol, bei dem
a) ein Benzol-haltiger Strom (a.1), der mindestens 90 Massen-%, bevorzugt mindestens 95 Massen-%, und besonders bevorzugt mindestens 99 Massen-% Benzol, jeweils bezogen auf die Gesamtmasse von (a.1), umfasst, in einem Reaktor mit einem Gemisch aus Schwefelsäure (a.2) und Salpetersäure (a.3) unter adiabaten Bedingungen umgesetzt wird, wobei Benzol in einem stöchiometrischen Überschuss bezogen auf Salpetersäure (a.3) von bevorzugt 2,0 % bis 20 %, besonders bevorzugt von 5,0 % bis 10 % der Theorie eingesetzt wird, und wobei die Menge M' des dem Reaktor pro Stunde zugeführten Benzol-haltigen Stroms (a.1) innerhalb eines Zeitraums t von Beginn der Nitrierung bis zum Erreichen eines vorgegebenen Soll-Werts für M' gesteigert wird,
b) das in Schritt a) erhaltene Verfahrensprodukt in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser zu einer wässrigen Phase (c.1) mit gegenüber (b.1) erhöhter Schwefelsäurekonzentration aufkonzentriert wird, und wobei die Phase (c.1) teilweise bis vollständig in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird, bevorzugt durch Waschen mit wässrigen Medien und anschließender Rektifikation, wobei ein Benzol-haltiger Strom (d.2) gewonnen wird (das sog. "Rückbenzol"), der bevorzugt 40,0 Massen-% bis 99,9 Massen-%, besonders bevorzugt 60,0 Massen-% bis 99,9 Massen-%, ganz besonders bevorzugt 80,0 Massen-% bis 99,9 Massen-% Benzol, jeweils bezogen auf die Gesamtmasse von (d.2), umfasst, und der teilweise bis vollständig in Schritt a) zurückgeführt und als Bestandteil von (a.1) eingesetzt wird, wobei die Rückführung von (d.2) während des Zeitraums t unterbleiben kann,
und bei dem
mindestens während des Zeitraums t dem Reaktor nur ein solcher Benzol-haltiger Strom (a.1) zugeführt wird, der einen Gehalt an aliphatischen organischen Verbindungen von kleiner als 1,5 Massen-%, bevorzugt von kleiner als 0,50 Massen-%, besonders bevorzugt von kleiner als 0,20 Massen-%, ganz besonders bevorzugt von kleiner als 0,10 Massen-%, jeweils bezogen auf die Gesamtmasse von (a.1), aufweist.

Der *auf Salpetersäure bezogene Benzolüberschuss* von 2,0 % bis 20 %, bevorzugt von 5,0 % bis 10 % der Theorie, bezieht sich auf das molare Verhältnis von Benzol und Salpetersäure. Theoretisch reagiert ein Mol Salpetersäure mit einem Mol Benzol zu einem Mol Nitrobenzol.

Dem Fachmann ist bekannt, dass ein kontinuierlich betriebener industrieller Prozess ausgehend von einer nicht in Betrieb befindlichen Produktionsanlage (z. B. nach einem wartungsbedingtem Stillstand) nicht augenblicklich wieder auf die Prozessparameter vor dem Produktionsstillstand hochgefahren werden kann. Edukte und Apparaturen müssen aufgeheizt werden, Apparate müssen ggf. inertisiert werden, die Belastung der Apparate mit den Edukten wird allmählich auf den angestrebten Soll-Wert gesteigert. Wenn eine Produktionsanlage zur Herstellung von Nitrobenzol bei einer *Soll*-*Belastung M'_{Soll}* von x [kg(Benzol)/h] betrieben werden soll, so kann diese Soll-Belastung beispielsweise erreicht werden, indem zu Beginn der Nitrierung die Belastung M' zunächst auf einen Wert von 0,25 x eingestellt und die Belastung dann über die Zwischenstufen M' = 0,50 x und M' = 0,75 x innerhalb von 4 Stunden auf den Wert M' = x = M'_{Soll} gesteigert wird. Alternativ kann auch von einem bestimmten Startwert aus, z. B. M' = 0,50 x, eine kontinuierliche Laststeigerung bis M' = x durchgeführt werden. Diese Beispiele stehen natürlich nur exemplarisch für eine Vielzahl möglicher Anfahrprozeduren, deren genaue Ausgestaltung von den konkreten Gegebenheiten einer Produktionsanlage abhängt und daher nicht verallgemeinert werden kann. Ein gemeinsames Merkmal aller denkbaren Anfahrprozeduren ist jedoch, dass erst nach Verstreichen eines Zeitraums t die angestrebte Soll-Belastung von x erreicht wird. Dieser Zeitraum t wird erfindungsgemäß als *Anfahrzeit* bezeichnet. Während der Anfahrzeit wird der dem Reaktor kontinuierlich zugeführte Mengenstrom an Salpetersäure (a.3) natürlich dem jeweiligen Mengenstrom an Benzol-haltigem Strom (a.1) angepasst, d. h., zu Beginn der Anfahrzeit, wenn dem Reaktor erst ein Bruchteil der angestrebten Soll-Belastung an Benzol M'_{Soll} zugeführt wird, wird dem Reaktor auch nur ein entsprechender Bruchteil an Salpetersäure zugeführt. Bevorzugt wird während der Anfahrzeit t der gleiche prozentuale Überschuss von Benzol bezogen auf Salpetersäure eingehalten wie nach Erreichen der Soll-Belastung M'_{Soll}. Das Massenverhältnis von Salpetersäure (a.3) zu Schwefelsäure (a.2) kann während der Anfahrzeit von demjenigen nach Erreichen der Soll-Belastung an Benzol M'_{Soll} abweichen; es kann insbesondere geringer sein. Insbesondere ist es bevorzugt, dem Reaktor zunächst nur Schwefelsäure (a.2) zuzuführen und erst nach Erreichen eines stabilen Betriebszustands des Schwefelsäurekreislaufs Salpetersäure (a.3) und Benzol-haltigen Strom (a.1) zuzuführen.

*Aliphatische organische Verbindungen* im Sinne der vorliegenden Erfindung umfassen bevorzugt Cyclohexan, Heptan, Methylcyclohexan, Bicycloheptan, Isomere des Dimethylcyclopentans, Ethylcyclopentan, Pentan, Cyclopentan und 2-Methylhexan. Erfindungsgemäß muss der Gehalt an aliphatischen organischen Verbindungen im Benzol-haltigen Strom (a.1) (dem sog. "Einsatzbenzol") kontrolliert werden. Zu diesem Zweck sind analytische Messungen erforderlich. Gemessen werden das Frischbenzol im Tank, das Einsatzbenzol und das Rückbenzol (d.2), und zwar bevorzugt durch Probenahme an den entsprechenden Stellen und Analyse der Proben mittels Gaschromatographie. Andere Bestimmungsmethoden (z. B. spektroskopische Methoden), ggf. auch online oder inline, können, wenn auch nicht bevorzugt, grundsätzlich ebenfalls angewandt werden. Maßgeblich für die erfindungsgemäße Obergrenze des Gehalts an organischen aliphatischen Verbindungen ist jedoch der durch Gaschromatographie ermittelte Wert. Die Analyse des Stroms (d.2) erfolgt in einer bevorzugten Ausführungsform, nämlich der Aufarbeitung mittels Rektifikation, am Kopf der Rektifikationskolonne. Das Wort *"ein"* ist im Rahmen dieser Erfindung im Zusammenhang mit zählbaren Größen nur dann als Zahlwort zu verstehen, wenn dies ausdrücklich gesagt wird. Bspw. schließt der Ausdruck *"ein Reaktor"* die Möglichkeit des Vorhandenseins mehrerer (in Reihe oder parallel geschalteter) Reaktoren nicht aus.

Erfindungswesentlich ist nun, dass der Gehalt an aliphatischen organischen Verbindungen im Benzol-haltigen Strom (a.1) (dem sog. "Einsatzbenzol") mindestens während der Anfahrzeit t den zuvor genannten Konzentrationen entspricht. Dieses Ziel kann auf alternative Weisen erreicht werden:
In einer ersten Variante erfolgt die teilweise bis vollständige Rückführung des Benzol-haltigen Stroms (d.2) auch schon während der Anfahrzeit. In diesem Fall ist das Einsatzbenzol während des gesamten Nitrierprozesses eine Mischung aus frischem Benzol technischer Reinheit (dem sog. "Frischbenzol") und dem zurückgeführten Benzol-haltigen Strom (d.2) (dem sog. "Rückbenzol"). Mindestens während der Anfahrzeit sind der Benzolüberschuss in Schritt a) und das Verhältnis von Frischbenzol zu Rückbenzol in Abhängigkeit insbesondere der Reinheit des Rückbenzols so einzustellen, dass der geforderte Höchstgehalt an aliphatischen organischen Verbindungen im Einsatzbenzol von kleiner als 1,5 Massen-%, bevorzugt von kleiner als 0,50 Massen-%, besonders bevorzugt von kleiner als 0,20 Massen-%, ganz besonders bevorzugt von kleiner als 0,10 Massen-%, jeweils bezogen auf die Gesamtmasse des Einsatzbenzols (a.1), nicht überschritten wird. Grundsätzlich ist natürlich auch die Reinheit des zur Verfügung stehenden Frischbenzols zu berücksichtigen. In der Regel steht dieses jedoch in einer solchen Reinheit zur Verfügung, dass das Hauptaugenmerk auf das Rückbenzol zu richten ist.

In einer zweiten Variante erfolgt die teilweise bis vollständige Rückführung des Benzol-haltigen Stroms (d.2) (dem sog. "Rückbenzol") erst nach Verstreichen der Anfahrzeit. In diesem Fall besteht das Einsatzbenzol während der Anfahrzeit aus frischem Benzol technischer Reinheit (dem sog. "Frischbenzol"), und erst nach Verstreichen der Anfahrzeit wird eine Mischung aus Frischbenzol und Rückbenzol als Einsatzbenzol verwendet. Diese Variante hat zwar den Nachteil, dass während der Anfahrzeit das Rückbenzol nicht in den Prozess rezykliert werden kann, ist jedoch technisch einfacher zu realisieren, weil das Abstimmen des Benzolüberschusses in Schritt a) und des Verhältnisses von Frisch- zu Rückbenzol in Abhängigkeit der Reinheit insbesondere des Rückbenzols unterbleiben kann. Da kommerziell erhältliches Frischbenzol in der Regel einen Gehalt an aliphatischen organischen Verbindungen aufweist, der den erfindungsgemäßen Anforderungen genügt, kann dieses meist direkt eingesetzt werden. Sollte jedoch nur solches Frischbenzol zur Verfügung stehen, dessen Reinheit den erfindungsgemäßen Anforderungen nicht genügt, so muss dieses vor seinem Einsatz in Schritt a) gereinigt werden, bevorzugt durch Destillation.

Nachstehend werden die beiden erfindungsgemäßen Varianten detailliert erläutert. Verschiedene Ausführungsformen innerhalb einer Variante sind dabei beliebig miteinander kombinierbar, solange sich für den Fachmann aus dem Kontext nicht eindeutig das Gegenteil ergibt.

### Variante 1

Abhängig von der Reinheit des zur Verfügung stehenden Frischbenzols und der Reinheit des Rückbenzols (d.2) werden beide in einem solchen Verhältnis gemischt, dass die erfindungsgemäßen Anforderungen des resultierenden Mischstroms (d. h., des Einsatzbenzols (a.1)) in Bezug auf den Gehalt an aliphatischen organischen Verbindungen eingehalten werden. Der Gehalt an aliphatischen organischen Verbindungen im Einsatzbenzol (a.1) ergibt sich additiv aus dem Gehalt an aliphatischen organischen Verbindungen des Frisch- und des Rückbenzols unter Berücksichtigung des Mengenverhältnisses dieser beiden Ströme. Je höher der Gehalt an aliphatischen organischen Verbindungen im Rückbenzol ist, desto geringer ist die Menge dieses Stroms, die mit dem Frischbenzol zur Herstellung des Einsatzbenzols vermischt werden kann (siehe Beispiele). Insbesondere wenn der Anteil an aliphatischen organischen Verunreinigungen im gewonnenen Rückbenzol (d.2) relativ hoch ist und darüber hinaus während der Anfahrzeit die Produktionsanlage noch nicht unter optimalen Bedingungen läuft, kann es vorkommen, dass nicht der gesamte Strom (d.2) zurückgeführt werden kann. In Variante 1 werden jedoch bevorzugt stets mindestens 25 Massen-%, besonders bevorzugt stets mindestens 50 Massen-% des Stroms (d.2) in Schritt a) zurückgeführt. Nicht zurückgeführte Anteile des Stroms (d.2) werden entweder verbrannt, nach destillativer Aufarbeitung wieder in den Prozess zurückgeführt oder bevorzugt in einem Rückbenzol-Puffertank zwischengelagert und nach Verstreichen der Anfahrzeit nach und nach in den Prozess zurückgeführt, und zwar bevorzugt so, dass auch nach der Anfahrzeit die oben genannten Bedingungen (maximal zulässiger Aliphatengehalt in Strom (a.1)) eingehalten werden. Zwingend erforderlich ist die Einhaltung der erfindungsgemäßen Anforderungen an die Reinheit des Einsatzbenzols jedoch nur während der Anfahrzeit.

### Variante 2

In dieser Variante wird während der Anfahrzeit nur Frischbenzol eingesetzt. Da dieses in der Regel die erfindungsgemäßen Reinheitsanforderungen erfüllt, sind keine besonderen Vorkehrungen erforderlich. Nur wenn das einzusetzende Frischbenzol wider Erwarten eine unzureichende Qualität aufweisen sollte, muss dieses vor seinem Einsatz gereinigt werden, bevorzugt durch Destillation. Das während der Anfahrzeit anfallende Rückbenzol (d.2) kann, wie in Variante 1 für die nicht zurückgeführten Anteile des Stroms (d.2) beschrieben, behandelt werden.

Nachstehend werden die Schritte der Erfindung, die für beide Varianten gleich sind, detailliert erläutert. Verschiedene Ausführungsformen sind dabei beliebig miteinander kombinierbar, solange sich für den Fachmann aus dem Kontext nicht eindeutig das Gegenteil ergibt.

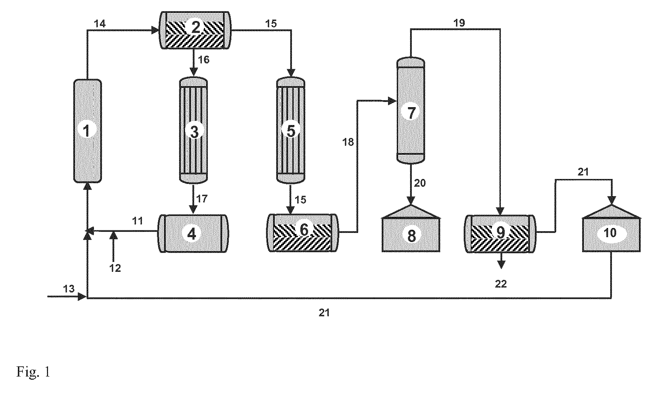

**Schritt a)** kann grundsätzlich nach allen aus dem Stand der Technik bekannten adiabaten Nitrierverfahren durchgeführt werden, solange mit diesen die spezifizierten Randbedingungen bzgl. des Benzolüberschusses und der Reinheit der Einsatzstoffe eingehalten werden können. Für die Ausführung dieses Schrittes des erfindungsgemäßen Verfahrens wird bevorzugt ein Rohrreaktor eingesetzt, in dem mehrere Dispergierelemente über die Länge des Reaktors verteilt angeordnet sind, die eine intensive Dispergierung und Durchmischung von Benzol, Salpetersäure und Schwefelsäure gewährleisten. Ein solcher Reaktor, sowie die Form einsetzbarer Dispergierelemente, sind beispielsweise in EP 0708 076 B1 (Figur 2) und EP 1 291 078 A2 (Figur 1) beschrieben. Bevorzugt wird Schritt a) in einer Verfahrensführung ausgeführt, wie sie in DE 10 2008 048 713 A1, insbesondere Absatz [0024], beschrieben ist.

Die Phasentrennung in **Schritt b)** erfolgt ebenfalls nach aus dem Stand der Technik an sich bekannten Verfahren in einem dem Fachmann bekannten Trennbehälter. Die wässrige Phase (b.1) enthält im Wesentlichen (infolge der Bildung von Reaktionswasser und durch das Einschleppen von Wasser in die Reaktion aus der eingesetzten Salpetersäure) verdünnte Schwefelsäure neben anorganischen Verunreinigungen, die organische Phase (b.2) enthält im Wesentlichen Nitrobenzol neben überschüssigem Benzol und organischen Verunreinigungen.

Die Aufkonzentrierung der wässrigen Phase (b.1) in **Schritt c)** erfolgt grundsätzlich wie aus dem Stand der Technik bekannt. Die Schwefelsäure in der wässrigen Phase wird in einem Entspannungsverdampfer (auch als Blitzverdampfer bezeichnet) aufkonzentriert, indem Wasser in einen Bereich reduzierten Druckes hinein verdampft wird. Bei richtiger Wahl der Reaktionsbedingungen in der adiabaten Nitrierung von Benzol mit Mischsäure erzielt man mit der Reaktionswärme der exothermen Reaktion eine so starke Erhitzung der Schwefelsäure enthaltenden wässrigen Phase (b.1), dass im Entspannungsverdampfer gleichzeitig wieder die Konzentration und Temperatur der Schwefelsäure enthaltenden wässrigen Phase eingestellt werden kann, die diese vor der Reaktion mit Benzol und Salpetersäure bei Eintritt in den Reaktorraum hatte, d. h., (c.1) entspricht hinsichtlich Temperatur und Konzentration (a.2). Dies ist beschrieben in EP 2 354 117 A1, insbesondere Absatz [0045].

Die Aufarbeitung der organischen Phase (b.2) in **Schritt d)** erfolgt grundsätzlich wie aus dem Stand der Technik bekannt. Eine bevorzugte Verfahrensweise wird nachstehend geschildert:
Die organische Phase (b.2), die üblicherweise noch Spuren an Säure enthält, wird in einer bis zwei Wäschen, bevorzugt einer Wäsche, mit einer wässrigen Waschflüssigkeit gewaschen und dann von der sauren wässrigen Phase durch Phasentrennung getrennt (bei mehreren Wäschen nach jeder einzelnen Wäsche). Bei diesem Vorgang werden die Säurereste, die das rohe Nitrobenzol (b.2) enthält, ausgewaschen; daher wird dieser Verfahrensschritt auch als *saure Wäsche* bezeichnet. Dieser Schritt ist aus dem Stand der Technik hinlänglich bekannt und wird daher hier nur kurz umrissen. Bevorzugt werden zur Durchführung dieser sauren Wäsche im Betrieb anfallende wässrige Ströme rezyklisiert. **(Schritt d(i).)**

Die so erhaltene organische Phase wird anschließend in einer bis zwei, bevorzugt einer alkalischen Wäsche(n) mit einer wässrigen Lösung einer Base, bevorzugt ausgewähltaus der Gruppe bestehend aus Natriumhydroxid, Natriumcarbonat und Natriumhydrogencarbonat, gewaschen und anschließend von dem alkalischen Waschwasser durch Phasentrennung getrennt (bei mehreren Wäschen nach jeder einzelnen Wäsche). Besonders bevorzugt wird als wässrige Baselösung Natronlauge eingesetzt. Dieser Schritt ist aus dem Stand der Technik hinlänglich bekannt und wird daher hier nur kurz umrissen. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt b) nicht vollständig entfernte Säurereste) in Schritt c) weitgehend bis vollständig, bevorzugt vollständig, neutralisiert werden. Die anschließende Aufarbeitung des alkalischen Abwassers kann nach den Verfahren des Standes der Technik, z. B. gemäß EP 1 593 654 A1 und EP 1 132 347 A2 erfolgen. **(Schritt d(ii).)**

Die so erhaltene organische Phase wird schließlich in mindestens einer, bevorzugt zwei bis vier, besonders bevorzugt zwei bis drei, ganz besonders bevorzugt zwei, neutralen Wäsche(n) mit Wasser gewaschen und anschließend von der wässrigen Phase durch Phasentrennung getrennt (bei mehreren Wäschen nach jeder einzelnen Wäsche). Dies kann grundsätzlich nach allen im Stand der Technik üblichen Verfahren geschehen. Als Waschwasser wird dabei bevorzugt vollentsalztes Wasser (VE-Wasser), besonders bevorzugt eine Mischung aus VE-Wasser und Dampfkondensat (d. i. ein Kondensat von Wasserdampf, welcher durch Wärmeaustausch von Wasser mit beliebigen exothermen Verfahrensschritten erhalten wurde) und ganz besonders bevorzugt Dampfkondensat eingesetzt. Bevorzugt ist eine Verfahrensweise, bei der in der letzten neutralen Wäsche eine Elektrophorese eingesetzt wird (siehe WO 2012/013678 A2). **(Schritt d(iii).)**

Das gewaschene Nitrobenzol wird schließlich von gelöstem Wasser, nicht umgesetztem Benzol und ggf. organischen Verunreinigungen durch weitere Aufarbeitung befreit. Diese Aufarbeitung erfolgt bevorzugt durch Destillation, wobei über Kopf die Brüden Wasser und Benzol und ggf. organische Verunreinigungen ausgetrieben werden. Die Brüden werden gekühlt und in einen Trennbehälter gefahren. In der unteren Phase setzt sich Wasser ab, das abgetrennt wird. In der oberen Phase befinden sich Benzol und Leichtsieder, die als Rückbenzol (d.2) wieder der Reaktion zugeführt werden. Bevorzugt wird als Destillationsapparat eine Rektifizierkolonne eingesetzt. Das Sumpfprodukt der Destillation wird, ggf. nach einer weiteren Destillation, in welcher Nitrobenzol *als Destillat* (also als Kopf- oder Seitenstromprodukt) erhalten wird, als Rein-Nitrobenzol (d.1) weiteren Anwendungen (wie der Hydrierung zu Anilin) zugeführt. **(Schritt d(iv).)**

Durch die erfindungsgemäßen Vorgehensweisen ergeben sich die folgenden Vorteile für das Anfahrprocedere der adiabaten Nitrierung:
i) Die Reaktionsmischung heizt sich schneller auf, weil der gewünschte Reaktionsumsatz schneller erreicht wird. Dadurch kann mit weniger Dampf gearbeitet werden, und es kann früher auf den reaktionsunterstützenden Einsatz von Dampf verzichtet werden.
ii) Die Benzolumsätze sind optimal und nur die gegenüber der theoretischen Menge überschüssige Menge an Benzol, nicht jedoch weiteres, durch unvollständige Reaktion vorhandenes Benzol belastet die Aufarbeitung in Schritt d).
iii) Die Bildung von Nebenprodukten in der Reaktion wie Pikrinsäure und Stickoxide (NOx) wird minimiert, weil stets ein Benzolüberschuss von bevorzugt 2,0 % bis 20 %, besonders bevorzugt von 5,0 % bis 10 % der Theorie eingesetzt wird.
iv) In der sog. "sauren Wäsche" wird die Entfernung von Säureresten aus dem Rohprodukt verbessert, weil die Phasentrennung nach der Wäsche infolge verringerten Ausgasens von organischen aliphatischen Verbindungen effizienter verläuft. Außerdem wird die Gefahr des Auftretens einer stabilen Emulsion, die sich nur schwierig in zwei Phasen trennen lässt, verringert.
v) Die Abwesenheit von aliphatischen organischen Verunreinigungen beim Anfahren der Nitrierung von Benzol hat außerdem den Vorteil, dass die hydraulische Belastung in der Reaktion geringer ausfällt und somit die Reaktion auch schneller auf Soll-Last hochgefahren werden kann.

Somit ermöglicht das erfindungsgemäße Verfahren durch Verwendung von Einsatzbenzol (a.1) mit einem Gehalt an aliphatischen organischen Verbindungen von kleiner als 1,5 Massen-%, bevorzugt von kleiner als 0,50 Massen-%, besonders bevorzugt von kleiner als 0,20 Massen-%, ganz besonders bevorzugt von kleiner als 0,10 Massen-%, jeweils bezogen auf die Gesamtmasse von (a.1), das Anfahren der adiabaten Nitrierung von Benzol und die anschließende Aufarbeitung des entstandenen Rohnitrobenzols in technisch reibungsloser Weise ohne Ausfallzeiten mit direkt hoher Endproduktqualität.

### Beispiele

Gehaltsangaben in ppm oder % sind Massenanteile bezogen auf die Gesamtmasse des jeweiligen Stoff(strom)s. Analysenwerte wurden, sofern nicht anders angegeben, mittels Gaschromatographie bestimmt.

### Allgemeine Bedingungen für die Herstellung von Nitrobenzol bei eingefahrener Produktionsanlage (also nach Verstreichen der Anfahrzeit t)

### (Siehe Figur 1)

Einem Reaktor (1) werden ein Schwefelsäure- (11), ein Salpetersäure- (12), ein Frischbenzol- (13) und ein Rückbenzolstrom (21) zugeführt. Es wird ein 5 bis 10%iger Überschuss an Benzol, bezogen auf Salpetersäure, eingesetzt. Von diesem Überschuss und der Qualität des Einsatzbenzols hängt die anfallende Menge an Rückbenzol ab. Nach vollständiger Umsetzung der Salpetersäure mit dem Benzol zu Nitrobenzol unter adiabater Reaktionsführung wird das nunmehr ca. 130 °C heiße Reaktionsprodukt (14) einem Phasentrennapparat (2) zugeführt, in dem das Reaktionsprodukt (14) in eine organische Phase ((15), = Roh-Nitrobenzol, enthaltend neben Nitrobenzol noch Benzol und Leichtsieder) und eine wässrige Phase ((16), = Absäure, enthaltend neben Schwefelsäure noch geringe Anteile an Nitrobenzol und Benzol) zerfällt. Die wässrige, hauptsächlich Schwefelsäure umfassende Phase (16) wird im Verdampfer (3) durch plötzliche Druckerniedrigung einer Blitzverdampfung von Wasser unterzogen und auf diese Weise aufkonzentriert. Die aufkonzentrierte Schwefelsäure (17) wird im Schwefelsäuretank (4) zur erneuten Verwendung gelagert. Nach dem Abtrennen im Phasentrennapparat wird das Rohnitrobenzol (15) in der Rohnitrobenzolkühlung (5) auf ca. 50 °C abgekühlt und der Wäsche (6) zugeführt. Der so erhaltene von Nitrophenolen und Salzen weitgehend befreite Strom gereinigten Roh-Nitrobenzols (18) wird wieder aufgeheizt und in einer Destillationskolonne (7) von Wasser, Benzol und anderen Leichtsiedern, welche über Kopf abgetrennt werden (19), befreit, wodurch getrocknetes Rein-Nitrobenzol (20) erhalten und in Tank (8) gelagert wird. Das kondensierte Kopfprodukt (19) der Destillationskolonne (7) wird einem Phasentrennapparat (9) zugeführt, in dem das Kopfprodukt in eine organische Phase ((21), enthaltend Benzol und Leichtsieder) und eine wässrige Phase (22) zerfällt. Die organische Phase (21) wird in einem Puffertank (10) zwischengelagert und von dort, wie oben schon beschrieben, zur Reaktion in den Zulauf des Reaktors (1) zurück gefahren.

So hergestelltes Nitrobenzol hat typischerweise eine Reinheit von ca.99,96 % (GC), einen Restbenzolgehalt von 0,0028 % (GC), einen Gehalt an 1,3-Dinitrobenzol von 0,0273 % (GC) und einen Nitrophenolgehalt von < 5 ppm (HPLC). Ferner weist das Nitrobenzol einen Wassergehalt von 0,0079 % (Karl-Fischer) auf.

### Allgemeine Bedingungen für das Anfahren eines adiabaten Nitrobenzol-Prozesses

### (Siehe Figur 1)

Die Kreislaufschwefelsäurepumpe wird gestartet, und Schwefelsäure aus dem Schwefelsäuretank (4) wird in den Reaktor (1) gefahren, läuft anschließend über in den Phasentrennapparat (2) und von dort in den Blitzverdampfer (3), um abschließend wieder im Schwefelsäuretank (4) anzukommen. Um schonend mit den in der Nitrierreaktion eingesetzten Schwefelsäurekreislaufpumpen umzugehen, wird beim Warmfahren der Anlage mit Schwefelsäure immer auch in Spuren Salpetersäure mitgefahren, um die Schwefelsäurekreislaufpumpe zu passivieren und die Zerstörung der Pumpe durch Korrosion zu verhindern. In kontinuierlicher Fahrweise wird die Schwefelsäure mit indirektem Dampf auf eine Temperatur von 101 °C aufgeheizt. Der Druck im Blitzverdampfer wird reduziert. Zum Anfahren der Nitrierung wird ein Benzolstrom (aus 13 (Frischbenzol) und gegebenenfalls 21 (Rückbenzol)) sowie der Salpetersäurestrom (12) zeitgleich zum Reaktoreingang gefahren, wo die Nitrierung mit der Dispergierung der Einsatzstoffe beginnt. Um die Nennkapazität der Anlage zu erreichen (M'_{Soll}), wird zunächst mit geringeren Mengenströmen an Benzol und Salpetersäure gestartet (in den Beispielen 1 bis 4 wurde die Anlage mit 50 % der Nennkapazität gestartet, was einer Produktionsleistung von 30 t/h (Nitrobenzol) entsprach). Diese Mengenströme werden dann während einer Anfahrzeit t auf die Nennlast erhöht. Das Hochfahren der Anlage kann manuell oder mit einer Anfahrautomatik gestaltet werden. Im Fall, dass während der Anfahrzeit nur mit Frischbenzol (Variante 2) oder mit Frischbenzol und einer reduzierten Menge an Rückbenzol gefahren wird, wird als Benzolüberschuss eine zusätzliche Menge an Frischbenzol gefahren. Die Anlage wurde jeweils so schnell wie möglich auf die Nennlast hochgefahren, wobei darauf geachtet wurde, einen vollständigen Umsatz der Salpetersäure zu erzielen.

**Tabelle 1: Gegenüberstellung der Ergebnisse aus den Beispielen**

| **Beispiel** | **Benzolüberschuss** | **Aliphatengehalt während der Anfahrzeit t** | | | **Verbrauch an 6 bar Dampf** | **Anfahrzeit** |
|---|---|---|---|---|---|---|
| | | **Frischbenzol** | **Rückbenzol** | **Einsatzbenzol** | | |
| **1 (Vergleich)** | 5,565 % | 788 ppm | 48 % | 4,43527 % | 4 t/h | Die Nitrierung sprang nicht an |
| **2 (Vergleich)** | 5,612 % | 993 ppm | 31 % | 2,38824 % | 3,1 t/h | 4h |
| **3 (erfindungsgemäß, Variante 1)** | 6,502 % | 975 ppm | 19 % | 1,49768 % | 1,4 t/h | 2h |
| **4 (erfindungsgemäß, Variante 2)** | 5,896 % | 1045 ppm | ohne Rückbenzol während t | 0,1045 % | 0,3 t/h | 2h |

Wie die Beispiele zeigen, lässt sich bei extrem hohen Aliphatengehalten im Einsatzbenzol die Reaktion nicht mehr anfahren (Beispiel 1). Aber auch wenn der Aliphatengehalt im Einsatzbenzol soweit erniedrigt wird wie in Beispiel 2, ist immer noch eine sehr lange Anfahrzeit von 4 h und ein erhöhter Dampfeintrag erforderlich, um Salpetersäure vollständig umzusetzen und so die Entstehung von NOₓ und Nebenprodukten wie Pikrinsäure zu vermeiden. Bei erfindungsgemäßer Vorgehensweise wird demgegenüber die Anfahrzeit und der Bedarf an Dampf signifikant reduziert.

## Patentansprüche

1. Kontinuierliches Verfahren zur Herstellung von Nitrobenzol durch Nitrierung von Benzol, bei dem
a) ein Benzol-haltiger Strom (a.1), der mindestens 90 Massen-% Benzol, bezogen auf die Gesamtmasse von (a.1), umfasst, in einem Reaktor mit einem Gemisch aus Schwefelsäure (a.2) und Salpetersäure (a.3) unter adiabaten Bedingungen umgesetzt wird, wobei Benzol in einem stöchiometrischen Überschuss bezogen auf Salpetersäure (a.3) eingesetzt wird, und wobei die Menge M' des dem Reaktor pro Stunde zugeführten Benzol-haltigen Stroms (a.1) innerhalb eines Zeitraums t von Beginn der Nitrierung bis zum Erreichen eines vorgegebenen Soll-Werts für M' gesteigert wird,
b) das in Schritt a) erhaltene Verfahrensprodukt in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser zu einer wässrigen Phase (c.1) mit gegenüber (b.1) erhöhter Schwefelsäurekonzentration aufkonzentriert wird, und wobei die Phase (c.1) teilweise bis vollständig in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird, wobei ein Benzol-haltiger Strom (d.2) gewonnen wird, der teilweise bis vollständig in Schritt a) zurückgeführt und als Bestandteil von (a.1) eingesetzt wird, wobei die Rückführung von (d.2) auch schon während des Zeitraums t erfolgt,
**dadurch gekennzeichnet, dass**
mindestens während des Zeitraums t dem Reaktor nur ein solcher Benzol-haltiger Strom (a.1) zugeführt wird, der einen Gehalt an aliphatischen organischen Verbindungen von kleiner als 1,5 Massen-%, bezogen auf die Gesamtmasse von (a.1), aufweist.

2. Kontinuierliches Verfahren zur Herstellung von Nitrobenzol durch Nitrierung von Benzol, bei dem
a) ein Benzol-haltiger Strom (a.1), der mindestens 90 Massen-% Benzol, bezogen auf die Gesamtmasse von (a.1), umfasst, in einem Reaktor mit einem Gemisch aus Schwefelsäure (a.2) und Salpetersäure (a.3) unter adiabaten Bedingungen umgesetzt wird, wobei Benzol in einem stöchiometrischen Überschuss bezogen auf Salpetersäure (a.3) eingesetzt wird, und wobei die Menge M' des dem Reaktor pro Stunde zugeführten Benzol-haltigen Stroms (a.1) innerhalb eines Zeitraums t von Beginn der Nitrierung bis zum Erreichen eines vorgegebenen Soll-Werts für M' gesteigert wird,
b) das in Schritt a) erhaltene Verfahrensprodukt in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser zu einer wässrigen Phase (c.1) mit gegenüber (b.1) erhöhter Schwefelsäurekonzentration aufkonzentriert wird, und wobei die Phase (c.1) teilweise bis vollständig in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird, wobei ein Benzol-haltiger Strom (d.2) gewonnen wird,
**dadurch gekennzeichnet, dass**
mindestens während des Zeitraums t dem Reaktor nur ein solcher Benzol-haltiger Strom (a.1) zugeführt wird, der einen Gehalt an aliphatischen organischen Verbindungen von kleiner als 1,5 Massen-%, bezogen auf die Gesamtmasse von (a.1), aufweist und dass
erst nach Verstreichen des Zeitraums t der in Schritt d) erhaltene Benzol-haltige Strom (d.2) teilweise bis vollständig in Schritt a) zurückgeführt und als Bestandteil von (a.1) eingesetzt wird.

3. Verfahren nach Anspruch 1 oder 2, bei dem die aliphatischen organischen Verbindungen ausgewählt sind aus der Gruppe bestehend aus Cyclohexan, Heptan, Methylcyclohexan, Bicycloheptan, Isomeren des Dimethylcyclopentans, Ethylcyclopentan, Pentan, Cyclopentan und 2-Methylhexan.

4. Verfahren nach Anspruch 1 oder 3, bei dem stets mindestens 25 Massen-% des Stroms (d.2) in Schritt a) zurückgeführt werden.

5. Verfahren nach einem der Ansprüche 1 bis 4, bei dem Benzol in Schritt a) in einem Überschuss von 2,0 % bis 20 % der Theorie eingesetzt wird.

6. Verfahren nach einem der Ansprüche 1 bis 5, bei dem der Benzol-haltige Strom (d.2) 40,0 Massen-% bis 99,9 Massen-% Benzol, bezogen auf die Gesamtmasse von (d.2), umfasst.

## Claims

1. A continuous process for the production of nitrobenzene by nitration of benzene, in which
a) a benzene-comprising stream (a.1), which encompasses at least 90 wt.% benzene, based on the total mass of (a.1), is reacted in a reactor with a mixture of sulfuric acid (a.2) and nitric acid (a.3) under adiabatic conditions, benzene being used in a stoichiometric excess based on nitric acid (a.3), and the quantity M' of the benzene-comprising stream (a.1) supplied to the reactor per hour being increased within a period t from the beginning of the nitration until a preset target value for M' is reached,
b) the process product obtained in step a) is separated into an aqueous phase encompassing sulfuric acid (b.1) and an organic phase encompassing nitrobenzene (b.2),
c) the aqueous phase (b.1) obtained in step b) is concentrated by evaporation of water to form an aqueous phase (c.1) with an increased sulfuric acid concentration compared with (b.1), and the phase (c.1) being partially to completely returned into step a) and used as a component of (a.2),
d) the organic phase (b.2) obtained in step b) is worked up to form pure nitrobenzene (d.1), a benzene-comprising stream (d.2) being obtained, which is partially to completely returned into step a) and used as a component of (a.1), the recirculation of (d.2) also already taking place during the period t,
**characterised in that**
at least during the period t only such a benzene-comprising stream (a.1) having a content of aliphatic organic compounds of less than 1.5 wt.%, based on the total mass of (a.1), is supplied to the reactor.

2. A continuous process for the production of nitrobenzene by nitration of benzene, in which
a) a benzene-comprising stream (a.1), which encompasses at least 90 wt.% benzene, based on the total mass of (a.1), is reacted in a reactor with a mixture of sulfuric acid (a.2) and nitric acid (a.3) under adiabatic conditions, benzene being used in a stoichiometric excess based on nitric acid (a.3), and the quantity M' of the benzene-comprising stream (a.1) supplied to the reactor per hour being increased within a period t from the beginning of the nitration until a preset target value for M' is reached,
b) the process product obtained in step a) is separated into an aqueous phase encompassing sulfuric acid (b.1) and an organic phase encompassing nitrobenzene (b.2),
c) the aqueous phase (b.1) obtained in step b) is concentrated by evaporation of water to form an aqueous phase (c.1) with an increased sulfuric acid concentration compared with (b.1), and the phase (c.1) being partially to completely returned into step a) and used as a component of (a.2),
d) the organic phase (b.2) obtained in step b) is worked up to form pure nitrobenzene (d.1), a benzene-comprising stream (d.2) being obtained,
**characterised in that**
at least during the period t only such a benzene-comprising stream (a.1) having a content of aliphatic organic compounds of less than 1.5 wt.%, based on the total mass of (a.1), is supplied to the reactor and that
only after the period t has passed is the benzene-comprising stream (d.2) obtained in step d) partially to completely returned into step a) and used as a component of (a.1).

3. The process according to claim 1 or 2, in which the aliphatic organic compounds are selected from the group consisting of cyclohexane, heptane, methylcyclohexane, bicycloheptane, isomers of dimethylcyclopentane, ethylcyclopentane, pentane, cyclopentane and 2-methylhexane.

4. The process according to claim 1 or 3, in which at least 25 wt.% of the stream (d.2) is always returned into step a).

5. The process according to one of claims 1 to 4, in which benzene is used in step a) in an excess of 2.0% to 20% of the theoretical value.

6. The process according to one of claims 1 to 5, in which the benzene-comprising stream (d.2) encompasses 40.0 wt.% to 99.9 wt.% benzene, based on the total mass of (d.2).

## Revendications

1. Procédé continu pour la préparation de nitrobenzène par nitruration de benzène, dans lequel
a) on met à réagir un flux (a.1) contenant du benzène, qui comprend au moins 90 % en masse de benzène, par rapport à la masse totale de (a.1), dans un réacteur avec un mélange d'acide sulfurique (a.2) et d'acide nitrique (a.3) dans des conditions adiabatiques, le benzène étant utilisé en un excès stoechiométrique par rapport à l'acide nitrique (a.3) et la quantité M' du flux (a.1) contenant du benzène introduit dans le réacteur par heure étant augmentée dans une période t depuis le début de la nitruration jusqu'à atteindre une valeur de consigne prédéterminée pour M',
b) on sépare le produit de procédé obtenu dans l'étape a) en une phase (b.1) aqueuse, contenant de l'acide sulfurique et une phase (b.2) organique, contenant du nitrobenzène,
c) on concentre la phase (b.1) aqueuse obtenue dans l'étape b) par évaporation de l'eau présentant une concentration en acide sulfurique augmentée par rapport à (b.1), la phase (c.1) étant recyclée en partie à en totalité dans l'étape a) et utilisée comme constituant de (a.2),
d) on transforme la phase (b.2) organique obtenue dans l'étape b) en nitrobenzène (d.1) pur, un flux contenant du benzène (d.2) étant obtenu, lequel est recyclé en partie à en totalité dans l'étape a) et est utilisé en tant que constituant de (a.1), la recirculation de (d.2) s'effectuant aussi déjà pendant la période t,
**caractérisé en ce**
**qu'**au moins pendant la période t, on alimente le réacteur uniquement en un flux (a.1) contenant du benzène qui présente une teneur en composés aliphatiques organiques inférieure à 1,5% en masse, par rapport à la masse totale de (a.1).

2. Procédé continu de préparation de nitrobenzène par nitruration de benzène, dans lequel
a) on met à réagir un flux (a.1) contenant du benzène, qui comprend au moins 90 % en masse de benzène, rapporté à la masse totale de (a.1), dans un réacteur avec un mélange d'acide sulfurique (a.2) et d'acide nitrique (a.3) dans des conditions adiabatiques, le benzène étant utilisé en un excès stoechiométrique par rapport à l'acide nitrique (a.3) et la quantité M' du flux (a.1) contenant du benzène introduit dans le réacteur par heure étant augmentée dans une periode t depuis le début de la nitruration jusqu'à atteindre une valeur de consigne prédéterminée pour M',
b) on sépare le produit de procédé obtenu dans l'étape a) en une phase (b.1) aqueuse, contenant de l'acide sulfurique et une phase (b.2) organique, contenant du nitrobenzène,
c) on concentre la phase (b.1) aqueuse obtenue dans l'étape b) par évaporation de l'eau en une phase (c.1) aqueuse présentant une concentration en acide sulfurique augmentée par rapport à (b.1), la phase (c.1) étant recyclée en partie à en totalité dans l'étape a) et utilisée comme constituant de (a.2),
d) on transfome la phase (b.2) organique obtenue dans l'étape b) en nitrobenzène (d.1) pur, un flux (d.2) contenant du benzène étant obtenu,
**caractérisé en ce**
**qu'**au moins pendant la période t, on achemine au réacteur uniquement un flux (a.1) contenant du benzène, qui présente une teneur en composés organiques aliphatiques inférieure à 1,5 % en masse, par rapport à la masse totale de (a.1), et en ce que
seulement après l'écoulement de la période t, le flux (d.2) contenant du benzène obtenu à l'étape d) est recyclé en partie à en totalité dans l'étape a) et est utilisé en tant que constituant de (a.1).

3. Procédé selon la revendication 1 ou 2, dans lequel les composés aliphatiques organiques sont choisis dans le groupe constitué par le cyclohexane, l'heptane, le méthylcyclohexane, le bicycloheptane, des isomères du diméthylcyclopentane, l'éthylcyclopentane, le pentane, le cylcopentane et le 2-méthylhexane.

4. Procédé selon la revendication 1 ou 3, dans lequel toujours au moins 25 % en masse du flux (d.2) sont recyclés dans l'étape a).

5. Procédé selon l'une quelconque des revendications 1 à 4, dans lequel le benzène est utilisé dans l'étape a) en un excès de 2,0% à 20% de la théorie.

6. Procédé selon l'une quelconque des revendications 1 à 5, dans lequel le flux (d.2) contenant du benzène comprend 40,0 % en masse à 99,9 % en masse de benzène, par rapport à la masse totale de (d.2).
